# EUROPÄISCHE PATENTSCHRIFT

(11) **EP 2 829 226 B1**
(45) Veröffentlichungstag und Bekanntmachung des Hinweises auf die Patenterteilung: **24.03.2021**
(21) Anmeldenummer: 13003716.1
(22) Anmeldetag: 24.07.2013
(51) Int. Cl.: A61B 5/042, A61M 1/36, A61B 5/0215

(54) **Perfusionskanüle mit integrierter Sensorik**
Perfusion cannula with integrated sensors
Canule de perfusion à technique sensorielle intégrée

(43) Veröffentlichungstag der Anmeldung: 28.01.2015
(73) Patentinhaber: novalung GmbH, 74076 Heilbronn (DE)
(72) Erfinder: Gorhan, Holger, 71101 Schönaich (DE); Hartig, Thomas, 74072 Heilbronn (DE)
(74) Vertreter: Graf von Stosch, Andreas

(56) Entgegenhaltungen:
- EP-B1- 0 127 476
- WO-A1-00/57805
- US-A- 5 029 585
- US-A- 5 065 010
- US-A- 5 425 724
- US-A- 5 433 742
- US-A1- 2002 111 662
- US-A1- 2010 094 255
- US-A1- 2011 092 955
- US-A1- 2011 295 177
- US-B2- 7 787 963

## Beschreibung

Die Erfindung betrifft eine Perfusionskanüle gemäß dem Oberbegriff des Anspruchs 1 zum Legen eines arteriellen oder venösen Zugangs, insbesondere in Verbindung mit einer Kreislaufersatztherapie.

Eine Kreislaufersatztherapie kann z.B. mittels eines extrakorporalen Lungen-Unterstützungssystems erfolgen, mittels welchem einem Patienten Blut oder ein das Blut ersetzendes Fluid an einer Stelle (insbesondere venös) entnommen wird und an einer anderen Stelle (insbesondere arteriell) wieder zugeführt wird, nachdem es mit Sauerstoff angereichert wurde und/oder der Anteil an Kohlenstoffdioxid darin gesenkt wurde. Hierzu können so genannte Membranventilatoren verwendet werden, welche auch als Membranoxygenatoren bezeichnet werden. Das Blut/Fluid kann mittels Kanülen entnommen bzw. zugeführt werden.

Mit dem Begriff "Perfusion" wird dabei die Versorgung von Organen mit Blut bezeichnet. Eine Organperfusion erfolgt auf natürliche Weise über Arterien und Venen, kann aber auch auf künstliche Weise erfolgen, z.B. über Bypässe oder extrakorporale Leitungen, die mittels Perfusionskanülen mit dem Organismus verbunden sein können. Perfusionskanülen werden häufig zur arteriellen Punktion eingesetzt, jedoch auch als venöse Perfusionskanülen. Sie stellen einen Versorgungskanal bereit, durch welche ein Fluid (z.B. Blut oder ein Blutersatzmittel) zu- oder abgeführt werden kann. Perfusionskanülen werden z.B. in Schocksituationen eines Menschen oder Tieres eingesetzt. In Schocksituationen ist dabei auch eine Überwachung des Vitalzustands von großer Bedeutung.

Um den Vitalzustand eines Menschen oder Tieres einschätzen zu können, ist es zum einen wichtig, die Herzfunktion zu prüfen. Dies kann durch elektrisches Erfassen der elektrischen Aktivität des Herzmuskels erfolgen. Hierbei kann ein Elektro-Kardiogramm erstellt werden, also eine Herzspannungskurve. Das Elektro-Kardiogramm wird üblicherweise über eine Impedanzmessung mittels auf der Haut des Menschen/Tieres (im Folgenden "Patient") angebrachter (Klebe-)Elektroden aufgezeichnet. Eine EKG-Messung wird dabei z.B. durch einen hohen Hautwiderstand erschwert oder durch Klebeelektroden, die nur unzureichend auf der Haut haften (insbesondere aufgrund feuchter Haut). Auch besteht die Gefahr, dass die Haut durch die Elektroden verletzt wird. Die Messung ist auch dadurch erschwert, dass die Elektroden verrutschen können, was bei Gegenwehr oder instabiler Lagerung eines Patienten ein großes Problem sein kann.

Eine weitere wichtige Messung betrifft den Blutdruck des Patienten. Eine Blutdruckmessung kann z.B. auf invasive Weise erfolgen, indem mittels einer Kanüle ein Druck in einer der peripheren Arterien des Patienten, z.B. einer Arm- oder Beinarterie, erfasst wird. Eine Druckmessung erfordert ein separates Druckmesssystem, in welchem der Druck über eine Wassersäule zu einem Druckmesssystem erfasst wird und einem Monitoringsystem zur Überprüfung von Vitalfunktionen bzw. zur Diagnose des Patienten zugeführt wird. Bei einer peripheren Vasokonstriktion (Gefäßverengung), insbesondere in einer Schocksituation des Patienten, kann die für diese Druckmessung erforderliche Kanülierung meist nur schwer gelegt werden, da die dafür bevorzugten Stellen an Handgelenken und Ellenbeugen stark verengt sind, oder da Leistengefässe durch extrakorporale Verfahren belegt sind.

US 5,029,585 A offenbart Elektroden zur Verwendung bei medizinischen Kathetern. Das Katheter weist eine Kanülenwand und ein von der Kanülenwand umgrenztes Perfusionsvolumen auf. Ein Sensorlumen ist vorgesehen. US 5,433,742 A beschreibt Herzkatheter, die adhäsive Bandelektroden aufweisen können. US 2002/111662 A1 beschreibt eine implantierbare medizinische Vorrichtung, die expandierbar ist und einen Drucksensor enthalten kann. Ein EKG-Sensor kann vorgesehen sein. US 2011/092955 A1 offenbart eine Perfusionskanüle mit einer ein Perfusionslumen umgrenzenden Kanülenwand. Ein Sensorlumen und eine Sensoreinrichtung können vorgesehen sein. Das Dokument US 2010/0094255 A1 beschreibt eine kanülierte Sensorvorrichtung mit einem proximalen und einem distalen Ende. WO 00/57805 A1 beschreibt eine Kanüle mit einem Drucksensor zur Messung des Flüssigkeitsdrucks.

Es ist eine Aufgabe der Erfindung, eine Perfusionskanüle bereitzustellen, welche die oben genannten Nachteile überwindet, insbesondere eine Perfusionskanüle mit verbesserter Funktionalität, z.B. in Schocksituationen eines zu therapierenden oder zu untersuchenden Patienten. Bevorzugt soll die Perfusionskanüle eine sichere Handhabung ermöglichen und/oder Funktionen auf besonders zuverlässige Weise erfüllen können. Weiter bevorzugt soll die Vorrichtung dabei auch robust im Hinblick auf Störeinflüsse sein.

Zumindest eine der zuvor genannten Aufgaben wird durch eine Perfusionskanüle gemäß Anspruch 1 gelöst.

Die Erfindung geht aus von einer Perfusionskanüle zum Legen eines Zugangs mit einer Kanülenwand und einem von der Kanülenwand umgrenzten Perfusionslumen, in welchem ein Fluidstrom die Perfusionskanüle durchströmen kann.

Erfindungsgemäß ist vorgesehen, dass die Perfusionskanüle ferner wenigstens ein Sensorlumen und wenigstens eine Sensoreinrichtung umfasst, welche zumindest teilweise in dem Sensorlumen angeordnet ist. Hierdurch kann mittels der Sensoreinrichtung auch ein Erfassen von Vitalparametern erfolgen, insbesondere eines (Blut-)Drucks und/oder einer Herzspannungskurve, wobei die Vitalparameter innerhalb des Körpers des Patienten erfasst werden können, insbesondere in direkter Umgebung des Herzens. Die Vitalparameter können dadurch selbst dann zuverlässig erfasst werden, wenn eine Schocksituation des Patienten vorliegt, in welcher eine Verengung (Vasokonstriktion) peripherer, distaler Gefäße des Patienten erfolgt.

Dabei ist nur eine einzige Kanülierung erforderlich, insbesondere eine femorale Kanülierung (im Oberschenkel- bzw. Leistenbereich). Mittels der Perfusionskanüle kann z.B. eine veno-arteriale Kreislaufunterstützung erfolgen, oder eine getriggerte diastolische Augmentation (Blutdruckerhöhung), oder allgemein eine Therapie in jeglicher Art von Schocksituationen (insbesondere kardiogen, spetisch, traumatisch), oder eine Reanimation. Dabei können simultan Vitalfunktionen überwacht werden.

Bevorzugt ist die Sensoreinrichtung ortsfest in Bezug auf ein (freies) distales Ende der Perfusionskanüle in dem Sensorlumen positioniert. Hierdurch wird dann die Position der Sensoreinrichtung relativ zum Patienten durch die Position der Perfusionskanüle insbesondere des distalen Endes hiervon vorgegeben. Besonders bevorzugt ist die Sensoreinrichtung in der Kanülenwand eingegossen oder darin eingebettet.

Als eine Perfusionskanüle ist dabei bevorzugt eine Vorrichtung zu verstehen, welche zum Legen eines Gefäß- oder Organzugangs für einen extrakorporalen Kreislauf ausgebildet ist und durch welche ein Fluid zu- oder abgeführt werden kann. Der Zugang kann dabei ein arterieller oder venöser Zugang sein, bevorzugt wird mit der Perfusionskanüle ein femoraler arterieller Zugang in einer Leistengegend des Patienten gelegt.

Als ein distales Ende ist dabei bevorzugt ein Ende der Perfusionskanüle zu verstehen, welches von einem Operateur weg weist und zu dem Patienten hinweist, also das Ende, welches in den Patienten eingebracht werden muss. Die Kanülengeometrie ist dabei bevorzugt so ausgelegt, dass möglichst kleine Übergänge an der Kanülenspitze vorhanden sind. Bevorzugt sind Übergänge kleiner als 0,1 mm.

Das Sensorlumen kann wahlweise als eine in einer Längsrichtung der Perfusionskanüle durchgehende Aussparung, Öffnung, Ausnehmung oder Bohrung ausgebildet sein oder auch im Bereich eines distalen Endes der Perfusionskanüle abgeschottet sein, insbesondere nachdem eine entsprechende Sensoreinrichtung in dem Sensorlumen in einer geeigneten Position ortsfest angeordnet wurde. Dabei kann auch die Abschottung selbst die Sensoreinrichtung in dem Sensorlumen fixieren und lagern. Bevorzugt ist das Sensorlumen im Querschnitt kreisrund ausgebildet. Hierdurch kann eine Sensoreinrichtung auf einfache Weise durch das Sensorlumen geführt werden, ohne mit Kanten oder Hinterschneidungen zu kollidieren. Wahlweise weist das Sensorlumen eine elliptische oder andere runde Innenmantelfläche mit stetigen Übergängen auf, insbesondere in Übereinstimmung mit einer Querschnittsgeometrie einer korrespondierenden Sensoreinrichtung.

Dabei können ein oder mehrere Sensorlumina jeweils über einen Tauch- und Drehvorgang zusammen mit der Kanülenwand hergestellt werden. Dies kann durch einen schichtweisen Auftrag von Polymer, vergleichbar mit einem Kerzentauchen, oder mittels einer Dosiereinrichtung auf einem rotierenden Werkstückträger erfolgen. Hierbei können z.B. ein Verstärkungsdraht, ein Kabel, oder andere Komponenten eingearbeitet werden.

Gemäß einer Variante ist das Sensorlumen durch einen in die Kanülenwand eingebrachten Hohlraum entsprechend einem Platzhalter beim Herstellungsprozess (z.B. Extrusion) gebildet. Hierdurch kann ein Sensorlumen an einer frei wählbaren Position in der Kanülenwand vorgesehen werden, und die Anzahl der Sensorlumina kann je nach Bedarf frei gewählt werden, insbesondere auch nachdem die Kanülenwand der Perfusionskanüle bereits hergestellt wurde. Ein Sensorlumen kann dabei im Zusammenhang mit Extrusion, oder mit einem Platzhalter, der nach der Herstellung entfernt wird, oder durch Einarbeiten von vorgefertigten Hohlkörpern hergestellt werden.

Gemäß einer bevorzugten Ausführungsform ist wenigstens ein Sensorlumen in die Kanülenwand integriert oder in radialer Richtung seitlich hervorstehend, insbesondere nach innen in das Perfusionslumen hervorstehend, an der Kanülenwand angeordnet. Durch ein integriertes Sensorlumen kann sichergestellt werden, dass die Funktion der Perfusionskanüle zum Entnehmen oder Zuführen des Fluids nicht beeinträchtigt wird. Durch ein nach innen in das Perfusionslumen hervorstehendes Sensorlumen kann sichergestellt werden, dass auch Sensoreinrichtungen, die im Durchmesser größer als die Wanddicke der Kanülenwand sind, in Verbindung der Persfusionskanüle genutzt werden können. Eine Anordnung seitlich innen an der Kanüle liefert den Vorteil einer einfachen Herstellbarkeit oder auch guter Strömungseigenschaften, insbesondere da die Kanülenwand dünner ausgeführt werden kann und damit höhere Durchflüsse bei einem bestimmten Kanülen-Außendurchmesser erzielt werden können.

Als ein in die Kanülenwand integriertes Sensorlumen ist dabei ein Lumen zu verstehen, welches innerhalb der Kanülenwand vorgesehen ist, ohne dass der Kanülenwand dafür eine besondere von der eigentlichen Kanülenwandgeometrie abweichende Geometrie verliehen wird. Ist die Kanülenwand z.B. als hohlzylindrischer Körper ausgebildet, so ist das Sensorlumen zwischen den beiden zylindrischen Mantelflächen vorgesehen, ohne dass von der zylindrischen Geometrie abgewichen werden muss. Ein in die Kanülenwand integriertes Sensorlumen weist damit einen Durchmesser auf, welcher kleiner ist als die Dicke der Kanülenwand.

Die Kanülenwand ist mit einer zumindest annähernd zylindrischen inneren Seite einer umlaufenden Innenmantelfläche ausgebildet. Weiter ist die Kanülenwand mit einer zumindest annähernd zylindrischen äußeren Seite einer umlaufenden Außenmantelfläche ausgebildet. Dies liefert, da beide Mantelflächen konzentrisch angeordnet sind, richtungsunabhängige Biegeeigenschaften und Steifigkeiten der Perfusionskanüle. Auch kann der Durchmesser der Perfusionskanüle bei dieser Querschnittsgeometrie auf einfache Weise entlang der Längserstreckung der Perfusionskanüle variabel ausgeführt sein, insbesondere bei einer sich verjüngenden Perfusionskanüle.

Gemäß einer bevorzugten Ausführungsform weist die Perfusionskanüle eine Einführlänge von mehr als 15 cm, bevorzugt mehr als 20 cm, weiter bevorzugt mehr als 25 cm, insbesondere im Bereich von 25 bis 50 cm auf. Hierdurch kann eine arterielle Perfusionskanüle bereitgestellt werden, welche auch in einem Leistenbereich eines Patienten eingebracht werden kann, und deren distales Ende bis in die Nähe des Herzens des Patienten gelangen kann.

Als Einführlänge ist dabei die Länge des Abschnitts der Perfusionskanüle zu verstehen, welcher maximal in einen Patienten eingeführt werden kann. Bei einer arteriellen Perfusionskanüle ist die Einführlänge bevorzugt deutlich größer als 25 cm und liegt z.B. im Bereich von 45 bis 50 cm. Zumindest über die Einführlänge weist die Perfusionskanüle bevorzugt eine Beschichtung mit guten Gleiteigenschaften an Gewebeoberflächen auf. Die Beschichtung kann eine Polyurethanbeschichtung sein.

Bevorzugt weist die Perfusionskanüle eine Gesamtlänge im Bereich von 20 bis 100, weiter bevorzugt 25 bis 75 cm auf. Als Gesamtlänge ist dabei die Länge der Perfusionskanüle von einem distalen Ende (Kanülenspitze) zu einem proximalen Ende zu verstehen, wobei die Perfusionskanüle an dem proximalen Ende mit einer Konsole, insbesondere einer Auswerteeinheit gekoppelt sein kann. Die Gesamtlänge ist größer als die Einführlänge, so dass ein Operateur die Perfusionskanüle jedenfalls handhaben kann, auch wenn sie über die maximale Einführlänge eingeführt ist. Bevorzugt ist die Gesamtlänge mindestens um 10 cm, bevorzugt mindestens 15 cm größer als die Einführlänge.

Bevorzugt ist die Perfusionskanüle dabei als arterielle Perfusionskanüle zum Einbringen in eine Arterie ausgebildet. Eine solche arterielle Perfusionskanüle weist bevorzugt keine radialen Aussparungen oder Löcher in der Kanülenwand auf, sondern lediglich am distalen Ende eine Öffnung, über welche einem Patienten Blut oder ein anderes Fluid zugeführt werden kann. Radiale Aussparungen oder Löcher in der Kanülenwand können jedoch bei venösen Perfusionskanülen vorgesehen sein, insbesondere um einem Patienten eine größere Menge an Blut oder Fluid pro Zeiteinheit entnehmen zu können (Erhöhung der Drainageleistung), insbesondere auch um eine entlastende Wirkung für das Herz bereitstellen zu können. Bei venösen Perfusionskanülen können z.B. zehn radiale Aussparungen oder Löcher vorgesehen sein, insbesondere in einem distalen Bereich der Perfusionskanüle, um Blut/Fluid direkt am Herzen entziehen zu können, insbesondere direkt aus dem Vorhof des Herzens oder der oberen Hohlvene (vena cava superior). Wahlweise weist auch die arterielle Perfusionskanüle ein oder zwei kleine radiale Löcher oder Aussparungen auf, aber deutlich weniger oder kleinere Löcher als eine venöse Perfusionskanüle. Hierdurch kann Blut nicht nur der Kanülenspitze, sondern auch seitlich davon zugeführt werden. Auch kann eine gute Durchblutung von Bauchorganen sichergestellt werden, und es können geringere Strömungsgeschwindigkeiten eingestellt werden. Die Löcher weisen bevorzugt einen Durchmesser von 0,5-5 mm, weiter bevorzugt 1-3 mm auf.

Gemäß einer bevorzugten Ausführungsform weist die Perfusionskanüle im Bereich eines distalen Endes der Perfusionskanüle einen Außendurchmesser zwischen 5 und 35 French (Fr) entsprechend etwa 1,7 und 11,7 mm, bevorzugt zwischen 12 und 25 French (Fr) entsprechend etwa 4 und 8,4 mm auf. Hierdurch kann eine besonders schmale, dünne und flexible Perfusionskanüle bereitgestellt werden, welche auch eine Diagnostikfunktion übernehmen kann. Der Patient wird mittels der erfindungsgemäßen Perfusionskanüle nicht stärker belastet als bei einer herkömmlichen Kanüle, insbesondere weil eine Inzision zum Einbringen der Perfusionskanüle trotz der zusätzlichen Sensoreinrichtung(en) klein bleiben kann.

Als Bereich eines distalen Endes ist dabei ein Abschnitt der Perfusionskanüle zu verstehen, welcher sich über eine Länge von maximal 5 cm, bevorzugt maximal 3 cm von einem distalen Ende der Perfusionskanüle in proximaler Richtung erstreckt.

Bevorzugt ist die Wandungsdicke der Perfusionskanüle entlang der gesamten Einführlänge unter 2 mm, bevorzugt unter 1 mm, weiter bevorzugt unter 0,5 mm. Hierdurch kann eine Perfusionskanüle, insbesondere eine arterielle Perfusionskanüle bereitgestellt werden, welche trotz einer bedeutenden Länge flexibel ist und leicht gehandhabt werden kann und von einer Leistengegend bis zu dem Herzen eines Patienten vorgeschoben werden kann.

Bevorzugt weist die Perfusionskanüle im Bereich eines proximalen Endes der Perfusionskanüle einen Außendurchmesser zwischen 5 und 35 French, bevorzugt zwischen 12 und 25 French auf. Hierdurch kann ein proximales Ende bereitgestellt werden, welches stabil ist, so dass eine Kanülenspitze auch über eine größere Einführlänge durch eine Arterie oder Vene geschoben werden kann. Bevorzugt verjüngt sich die Perfusionskanüle im Einführabschnitt, insbesondere kontinuierlich über die Einführlänge. Die Verjüngung ist dabei bevorzugt in einem Konnektorbereich der Perfusionskanüle vorgesehen oder besonders stark ausgeprägt. Der Außendurchmesser ist im Bereich der Einführlänge bevorzugt konstant. Eine Verjüngung liegt bevorzugt an der Kanülenspitze vor.

Gemäß einer bevorzugten Ausführungsform ist wenigstens eine der wenigstens einen Sensoreinrichtung als EKG-Sensor ausgebildet. Das (EKG-)Sensorlumen kann einen Durchmesser zwischen 0,05 und 2 mm, bevorzugt 0,2 und 0,5 mm aufweisen. Der EKG-Sensor kann dabei im Wesentlichen durch zwei Elektroden und die Elektroden elektrisch kontaktierende Leitungen gebildet sein. Hierdurch kann eine EKG-Messung mittels eines besonders kleinen Sensors erfolgen, der in dem Sensorlumen geführt werden kann. Ein Innendurchmesser des Sensorlumens, welcher auf den Außendurchmesser des EKG-Sensors abgestimmt ist, erleichtert die Montage des EKG-Sensors in der Perfusionskanüle. Es kann z.B. verhindert werden, dass der EKG-Sensor im Sensorlumen verkantet. Dabei kann eine dünne Kanülenwand mit guter Flexibilität (mit guten elastischen Eigenschaften) bereitgestellt werden, die trotz des Sensorlumens eine gute Stabilität aufweist. Bei einer geringen Wandstärke entsteht nur ein geringer Druckverlust.

Eine solche Perfusionskanüle mit einem EKG-Sensor kann für eine arterielle EKG-Messung vorgesehen sein, also eine EKG-Messung, bei welcher die Perfusionskanüle durch eine Arterie bis in die Nähe des Herzens eines Patienten eingebracht wird, insbesondere über die Leistengegend, ohne dass z.B. ein Brust- oder Bauchbereich des Patienten chirurgisch geöffnet werden muss. Der EKG-Sensor ist bevorzugt bipolar messend ausgebildet. Der EKG-Sensor kann mit einem proximalen Ende an eine Auswerteeinheit angeschlossen sein, z.B. über eine zugsichere Steckverbindung.

Gemäß einer bevorzugten Ausführungsform ist die wenigstens eine der wenigstens einen Sensoreinrichtung als EKG-Sensor ausgebildet und weist eine Ringelektrode auf, die außen um die Kanülenwand angeordnet ist und über eine Durchführung in der Kanülenwand mittels eines in dem entsprechenden Sensorlumen geführten elektrischen Leiters elektrisch kontaktiert ist. Hierdurch kann ein EKG-Sensor bereitgestellt werden, mittels welchem die Elektroden besonders nah in den Bereich des Herzens gebracht werden können. Dies ermöglicht eine genaue Messung. Die Materialwahl für die Perfusionskanüle kann weitgehend unabhängig von der elektrischen Funktion der Elektroden erfolgen. Die Elektroden können gegenüber der Kanüle elektrisch isoliert sein. Ferner ist die Messung unabhängig von einer Verdrehung der Perfusionskanüle um eine Mittenlängsachse der Perfusionskanüle, da die Ringelektroden umlaufend vorgesehen sind und in allen Richtungen einer Ebene senkrecht zu der Mittenlängsachse eine Messung durchführen können. Hierdurch ist die Handhabung der EKG-Messung besonders einfach und sicher. Eine zuverlässige Messung kann jedenfalls sichergestellt werden, unabhängig von dem Zustand des Patienten oder der Ausrichtung der Perfusionskanüle. Die Elektrode besteht bevorzugt aus einem biokompatiblen Material.

Die Ringelektrode kann unmittelbar mit einer elektrischen Leitung verbunden sein, welche durch das Sensorlumen geführt ist und mit einer Auswerteeinheit verbindbar ist. Die elektrische Leitung kann z.B. ein 3-5 poliges Kabel sein. Wahlweise kann die Ringelektrode auch mit einem weiteren Teil der EKG-Sensoreinrichtung gekoppelt sein, welche wiederum mit der Auswerteeinheit gekoppelt ist, insbesondere über eine elektrische Leitung. Die elektrische Leitung kann in dem Sensorlumen eingebettet sein.

Die Elektrode bzw. Ringelektrode ist bevorzugt eine passive Elektrode, die mit einer Leitung (einer von der Elektrode wegführenden Leitung) gekoppelt ist. Es können z.B. 3-5 polige Leitungen bzw. Kabel vorgesehen sein.

Bevorzugt weist die Perfusionskanüle zwei Elektroden oder Ringelektroden auf, welche in einem vorgegebenen Abstand zueinander angeordnet sind, insbesondere im Bereich von 5 bis 200 mm, bevorzugt von 30 bis 80 mm. Hierdurch kann eine Herzspannungskurve mit großer Genauigkeit aufgenommen werden, insbesondere da die Elektroden immer in einem vordefinierten Abstand zueinander angeordnet sind. Eine Fehlbedienung, wie z.B. beim Aufkleben von Elektroden auf den Brustkorb in nicht optimalen Positionen, kann vermieden werden.

Gemäß einer bevorzugten Ausführungsform ist die wenigstens eine der wenigstens einen Sensoreinrichtung als EKG-Sensor mit zwei innerhalb des Sensorlumens angeordneten Elektroden ausgebildet. Hierdurch kann eine besonders einfach herzustellende Perfusionskanüle bereitgestellt werden. Elektrische Durchführungen nach außen sind nicht erforderlich. Die Perfusionskanüle kann dabei mit einer zumindest im Wesentlichen ebene Außenmantelfläche ausgeführt sein, welche keine Kanten, Erhebungen oder Unstetigkeiten aufweist, so dass die Perfusionskanüle zum einen in Bezug auf ihre Mittenlängsachse gleiche Biegeeigenschaften aufweist, zum anderen auch mit geringem Widerstand in einer Arterie oder Vene verschoben werden kann.

Bevorzugt sind die Elektroden bzw. Ringelektroden in einem vorgegebenen Abstand zu einem distalen Ende der Perfusionskanüle positioniert. Hierdurch kann eine Positionierung der Elektroden über eine Position des distalen Endes der Perfusionskanüle erfolgen. Das distale Ende kann dabei in dem Aortenbogen der thorakalen Aorta (im Brustkorbbereich) angeordnet werden, wobei der Abstand der Elektroden von dem distalen Ende derart festgelegt ist, dass die Elektroden in einer messtechnisch zweckdienlichen Position in Bezug auf das Herz angeordnet werden.

Wenigstens eine der wenigstens einen Sensoreinrichtung ist als Drucksensor ausgebildet. Bevorzugt sind dabei mindestens zwei Sensorlumina vorgesehen und in dem anderen Sensorlumen ist ein EKG-Sensor angeordnet. Hierdurch ist eine schnelle und effiziente Erfassung von mehreren Vitalparametern möglich, und dabei ist nur ein einziger Gefäßzugang erforderlich, insbesondere ein arterieller Zugang. Die Perfusionskanüle kann sowohl zum Erfassen einer Herzspannungskurve als auch eines Blutdrucks verwendet werden. Dabei ist für die Erfassung von mindestens zwei Vitalparametern auch nur eine einzige Kanülierung erforderlich. Die Gefahr von Störeinflüssen kann verringert werden, insbesondere weil in vivo gemessen werden kann. Auch können weitere Vitalparameter, insbesondere ein Blutdruck, in direkter Umgebung des Herzens erfasst werden. Die Kanüle kann dabei (insbesondere zeitgleich) zum einen für die Messung der Vitalparameter, zum anderen aber auch ohne besondere Einschränkung als Perfusionskanüle für den extrakorporalen Kreislauf eingesetzt werden. Die Kanülenwand ist lediglich etwas dicker ausgeführt als bei einer herkömmlichen Perfusionskanüle, und ist möglicherweise etwas steifer.

Erfindungsgemäß ist das Sensorlumen zur Aufnahme des Drucksensors in die Kanülenwand integriert. Dabei können nicht nur zwei, sondern auch noch weitere Sensorlumina vorgesehen sein, z.B. drei bis fünf Sensorlumina.

Gemäß einer bevorzugten Variante sind die Sensorlumina konzentrisch um eine Mittenlängsachse der Perfusionskanüle angeordnet. Hierdurch kann eine Perfusionskanüle bereitgestellt werden, bei welcher die Auswahl eines bestimmten Sensorlumens für einen bestimmten Sensor ohne Einfluss auf die Messung ist, d.h., bei einer Messung muss eine Anordnung des Sensors in der Kanüle relativ zu der Kanülenwand nicht berücksichtigt werden, insbesondere weil die Lumina in demselben Abstand zu einer Außenmantelfläche der Perfusionskanüle angeordnet sind. Dies erleichtert auch eine Herstellung der Perfusionskanüle, insbesondere bei einer großen Anzahl von Sensorlumina, z.B. fünf Sensorlumina. Bevorzugt sind die Sensorlumina in einem gleichbleibenden Abstand zueinander über den Umfang verteilt angeordnet und dabei auf den gesamten Umfang bezogen symmetrisch angeordnet. Dadurch kann eine Perfusionskanüle bereitgestellt werden, welche in allen Raumrichtungen dieselbe Biegesteifigkeit um ihre Mittenlängsachse aufweist, zumindest wenn die Sensorlumina denselben (Innen-)Durchmesser aufweisen, und dadurch leichter gehandhabt werden kann.

Bevorzugt ist die Kanülenwand im Querschnitt ringförmig ausgebildet und weist eine Breite bzw. Wandstärke unter 2 mm, bevorzugt unter 1 mm, weiter bevorzugt unter 0,5 mm auf. Hierdurch kann eine Perfusionskanüle mit einer möglichst schmalen Kanülenwand bereitgestellt werden, welche flexibel ist und auch in alle Raumrichtungen auf dieselbe Weise gebogen werden kann.

Gemäß einer bevorzugten Ausführungsform ist der Drucksensor als faseroptischer Drucksensor ausgebildet. Hierdurch kann ein besonders kompakter Drucksensor bereitgestellt werden, der auf zweckdienliche Weise in ein kleines Lumen einer Perfusionskanüle integrierbar ist, ohne dass die Handhabung der Perfusionskanüle spürbar erschwert wird. Die Messung kann ohne Flüssigkeiten erfolgen, sie kann mit hoher Messgenauigkeit erfolgen, und die Messung ist wenig fehleranfällig. Wahlweise kann eine Messung über eine Wassersäule erfolgen.

Bevorzugt ist wenigstens ein Sensorlumen für einen faseroptischen Drucksensor vorgesehen und als beidseitig offener Kanal ausgebildet. Der Drucksensor kann z.B. in einer Einbettung (insbesondere einem transparenten Polymer) gelagert sein, welche auch eine Abdichtung sicherstellen kann. Wahlweise kann auch allein oder zusätzlich ein Schott durch einen Stopfen oder eine Wandung aus einem elastischen Material wie z.B. Silikon gebildet sein, wobei der Stopfen bzw. die Wandung im Bereich des distalen Endes der Sensoreinrichtung vorgesehen sein kann.

Bevorzugt ist wenigstens ein Sensorlumen für einen möglichst kleinen, optisch messenden, faserbasierten Sensor vorgesehen, z.B. für einen Drucksensor des kanadischen Unternehmens FISO Technologies. Solche Sensoren können einen Durchmesser im Bereich von nur 200 µm oder noch weniger aufweisen. Sie erfordern keine Wassersäule und auch keine separate Halteplatte. Sie sind z.B. aus einem Glasmaterial hergestellt und können biokompatibel ausgeführt sein. Der Drucksensor selbst ist bevorzugt an der Spitze der Sensoreinrichtung angeordnet. Druckunterschiede oder Druckänderungen können sehr schnell und mit guter Genauigkeit erfasst werden.

Bevorzugt weist das Druck-Sensorlumen einen Durchmesser im Bereich von 0,3 bis 0,8 mm, bevorzugt 0,4 bis 0,7 mm, besonders bevorzugt 0,5 bis 0,6 mm, insbesondere 0,55 mm auf.

Gemäß einer bevorzugten Ausführungsform weist der faseroptische Drucksensor ein distales Ende auf, welches in einem Abstand von maximal 5 cm, bevorzugt maximal 3 cm, weiter bevorzugt maximal 1 cm von einem distalen Ende der Perfusionskanüle angeordnet ist. Hierdurch kann die Druckmessung an einem über die Position des distalen Endes der Perfusionskanüle vorgebbaren Punkt erfolgen. Wahlweise kann eine seitliche Durchführung durch die Kanülenwand erfolgen, z.B. wie bei Durchführungen für die elektrischen Leitungen zu Ringelektroden.

Gemäß einer bevorzugten Ausführungsform ist wenigstens eine der wenigstens einen Sensoreinrichtung fluiddicht im entsprechenden Sensorlumen eingebettet, insbesondere im Bereich eines distalen Endes der Sensoreinrichtung, bevorzugt auch im Bereich eines proximalen Endes der Sensoreinrichtung. Hierdurch kann sichergestellt werden, dass die Sensoreinrichtung in der Perfusionskanüle nicht verrutscht oder tordiert wird. Ferner müssen das bzw. die Sensorlumen/-lumina auch nicht vor jeder Verwendung sterilisiert werden. Die Einbettung kann z.B. aus Silikon, Silikon-Polyurethan Copolymer, PVC und/oder Polyurethan bestehen. Eine Abdichtung kann dabei durch die Einbettung selbst sichergestellt werden.

Gemäß einer bevorzugten Ausführungsform weist die Perfusionskanüle eine Drahtverstärkung auf, die vorzugsweise radial außen von dem wenigstens einen Sensorlumen angeordnet ist. Hierdurch kann der Perfusionskanüle eine höhere (Knick-)Stabilität verliehen werden, und die Gefahr eines Bruchs der Perfusionskanüle aufgrund zu starker Biegung kann vermindert werden. Eine Drahtverstärkung ist bei weniger knickstabilen Materialien bzw. Kanülen zweckdienlich.

Bevorzugt erstreckt sich die Drahtverstärkung von einem proximalen Ende der Perfusionskanüle bis zu einer Ringelektrode, oder zu einer innerhalb der Kanülenwand angeordneten und aus Sicht des Operateurs ersten Elektrode. Weiter bevorzugt wird die Kanülenwand mit der Drahtverstärkung umwickelt und dann mit einer Beschichtung versehen. Die Drahtverstärkung ist dabei bevorzugt komplett eingebettet, insbesondere in einem Polymer.

Gemäß einer bevorzugten Ausführungsform ist die Perfusionskanüle mit einer Schicht Polyurethan überzogen. Hierdurch können die Gleiteigenschaften der Perfusionskanüle in einem Gefäß bzw. einer Arterie oder Vene verbessert werden. Bevorzugt ist auch die Innenfläche eines Sensorlumens mit einer Beschichtung versehen, insbesondere Polyurethan. Bevorzugt weist die Perfusionskanüle eine Innenschicht Polyurethan auf, um welche eine Drahtverstärkung vorgesehen ist, die von einer Außenschicht Polyurethan umgeben ist. Das Material Polyurethan kann dabei auch ersetzt werden durch Silikon, Silikon-Polyurethan Copolymer oder PVC.

Bevorzugt ist die Perfusionskanüle mit weiteren Schichten, insbesondere Polyurethanschichten oder Schichten aus Silikon, Silikon-Polyurethan Copolymer oder PVC, überzogen. Bevorzugt ist auch eine Drahtverstärkung mit mindestens einer weiteren Schicht überzogen.

Gemäß einer bevorzugten Ausführungsform besteht die Perfusionskanüle im Wesentlichen aus Silikon, Silikon-Polyurethan Copolymer, PVC, Polyurethan (PUR) und/oder nichtrostendem VA-Stahl. Hierdurch kann eine gute Flexibilität und Beständigkeit sichergestellt werden.

Bevorzugt ist die Kanülenwand einstückig aufgebaut, also aus einem integralen Körper aus demselben Material, abgesehen von etwaigen Beschichtungen. Hierdurch kann eine robuste, in allen Raumrichtungen zumindest annähernd auf dieselbe Weise belastbare Perfusionskanüle bereitgestellt werden. Ferner kann sichergestellt werden, dass keine Hohlräume vorliegen, in welchen Gas eingeschlossen sein könnte, was das Risiko einer Embolie mit sich bringen könnte.

Bevorzugt sind das Perfusionslumen und das/die Sensorlumen in Vollmaterial eingebracht und in die Kanülenwand integriert. Hierdurch kann eine Perfusionskanüle bereitgestellt werden, die sich leicht handhaben lässt und mit einer zylindrischen Außenmantelfläche ausgeführt sein kann.

Gemäß einer bevorzugten Ausführungsform weist die Perfusionskanüle ein freies Arbeitslumen auf, in welchem eine Arbeitssonde, z.B. eine Diagnostikeinrichtung in Form eines Diagnostik-Katheters, anordenbar ist. Hierdurch kann die Funktionalität der Perfusionskanüle noch erweitert werden. Ein freies Arbeitslumen ist dabei eine Art Arbeitskanal, in welchem wahlweise, insbesondere im Hinblick auf bestimmte Anwendungen, z.B. eine Diagnostikeinrichtung eingebracht sein kann. Das Arbeitslumen kann je nach Bedarf eines Kunden vorgesehen werden und wahlweise auch bereits eine darin angeordnete oder eingebettete Arbeitssonde aufweisen. Zunächst ist das Arbeitslumen jedoch frei, also leer ohne irgendeine darin angeordnete Einrichtung. Das Arbeitslumen weist bevorzugt einen Durchmesser von 1 bis 6 French (Fr) entsprechend 0,35 bis 2 mm, weiter bevorzugt 4 Fr entsprechend 1,35 mm auf. Das Arbeitslumen kann mittels eines Ventils gegenüber dem distalen Ende der Perfusionskanüle abgeschottet sein. Das Arbeitslumen kann als kollabierendes Lumen ausgebildet sein bzw. ein funktionsloses Mandrel aufweisen. Ein kollabierendes Lumen ist ein Lumen, welches bei Einführung der Kanüle durch einen Dilatator der Kanüle zur Seite geschoben werden kann, und welches sich bei Entfernung des Dilatators entfalten kann. Unter einem Mandrel ist ein Platzhalter zu verstehen, welcher in einem Arbeitslumen angeordnet werden kann, um dieses zu schließen bzw. zu füllen, wenn das Arbeitslumen keine Verwendung findet. Das Mandrel kann dadurch eine Verklottung des Arbeitslumens verhindern. Das Mandrel kann aus einem langem Kunststoffrohling bestehen, welches einen Außendurchmesser entsprechend dem Innendurchmesser des Arbeitslumens aufweist.

Bevorzugt weist die Perfusionskanüle mindestens drei Lumina auf, von denen zwei als Sensorlumina mit einer darin angeordneten Sensoreinrichtung ausgebildet sind und ein Lumen als Arbeitslumen mit der darin anordenbaren Arbeitssonde ausgebildet ist, wobei die Lumina konzentrisch und in demselben Abstand zueinander um die Mittenlängsachse der Perfusionskanüle angeordnet sein können.

Eine erfindungsgemäße Perfusionskanüle kann Teil eines Systems sein, welches eine Auswerteeinheit aufweist. In dem erfindungsgemäßen System, insbesondere einem Kreislauf-Unterstützungssystem, sind eine erfindungsgemäße Perfusionskanüle für einen arteriellen Zugang und eine Drainagekanüle vorgesehen, wobei das System eine Auswerteeinheit zum Auswerten von Messwerten zumindest einer der jeweils wenigstens einen Sensoreinrichtung der erfindungsgemäßen Perfusionskanüle aufweist.

Das System weist bevorzugt eine arterielle Perfusionskanüle für das Zuführen eines Fluids auf und eine venöse Perfusionskanüle für das Entnehmen eines Fluids auf. Hierbei kann z.B. eine EKG-Messung mit einer der Perfusionskanülen erfolgen, und eine Druckmessung mit einer anderen der Perfusionskanülen erfolgen. Wahlweise kann in beiden Fällen eine Druckmessung und eine EKG-Messung erfolgen, also jeweils an zwei unterschiedlichen Stellen im Körper des Patienten. Bevorzugt erfolgt eine EKG-Messung arteriell. Bevorzugt ist die arterielle Perfusionskanüle dazu ausgebildet, in einem Leistenbereich eines Patienten eingeführt und bis zum Herzen des Patienten vorgeschoben zu werden und weist eine Einführlänge auf, die im Bereich von 25 bis 60 cm liegt, bevorzugt über 45 cm.

Das System, speziell ein Lungen-Unterstützungssystem, kann z.B. auch einen Oxygenator aufweisen, mittels welchem einem dem Patienten zugeführten Fluid oder Blut Sauerstoff hinzugefügt werden kann.

Mit einer erfindungsgemäßen Perfusionskanüle kann ein Patient insbesondere auf die folgende Weise untersucht und/oder therapiert werden:
Zunächst wird ein Zugang in einem Leistenbereich des Patienten gelegt. Dann erfolgt ein Einführen der Perfusionskanüle durch eine Arterie bis hin zu dem Herzen des Patienten. Daraufhin erfolgt eine Freigabe des Perfusionslumens und eine Zufuhr eines Fluids. Zeitgleich mit der Zufuhr kann eine Messung des EKG und/oder eine Messung eines Blutdrucks erfolgen

Die Erfindung wird definiert durch eine Vorrichtung nach Anspruch 1.

In den nachfolgenden Figuren wird die Erfindung anhand von Ausführungsbeispielen näher erläutert, wobei unterschiedliche Anordnungen von Sensorlumina gezeigt sind. Es zeigen:
- Fig. 1: in einer schematischen Ansicht ein Lungen-Unterstützungssystem mit einer schematisch dargestellten Perfusionskanüle gemäß einem Beispiel;
- Fig.2: in einer schematischen Querschnittansicht eine Perfusionskanüle gemäß einem Beispiel;
- Fig.3: in einer schematischen Längsschnittansicht eine Perfusionskanüle (nicht beansprucht);
- Fig.4: in einer schematischen Längsschnittansicht eine Perfusionskanüle gemäß noch einem weiteren Beispiel;
- Fig.5: in einer schematischen Längsschnittansicht eine Perfusionskanüle gemäß noch einem weiteren Beispiel; und
- Fig.6: in einer schematischen Querschnittansicht eine Perfusionskanüle gemäß einem Ausführungsbeispiel der Erfindung.

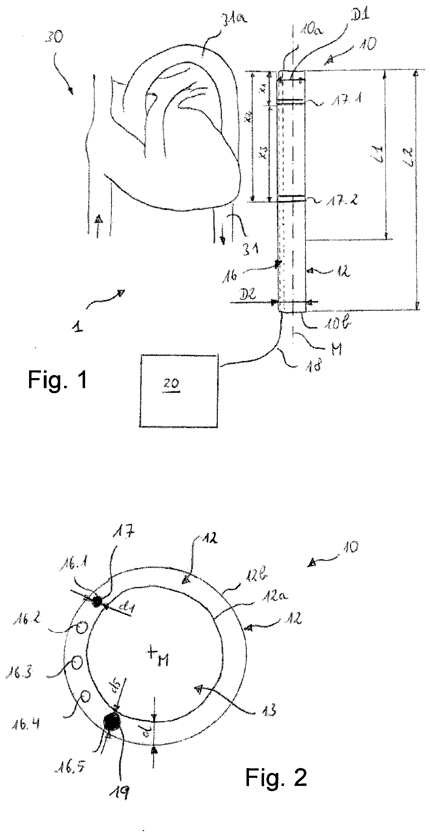

In der Fig. 1 ist ein Lungen-Unterstützungssystem 1 mit einer Perfusionskanüle 10 gezeigt, welche im Bereich eines Herzens 30 eines Patienten, insbesondere in einer Aorta 31 nahe eines Aortenbogens 31a angeordnet werden kann. Die Perfusionskanüle 10 ist über eine elektrische Verbindung 18 mit einer Auswerteeinheit 20 gekoppelt und erstreckt sich entlang einer Mittenlängsachse M. Sie weist ein distales Ende 10a (Kanülenspitze) und ein proximales Ende 10b auf. Die Perfusionskanüle 10 ist in zwei Abschnitte unterteilt, einen distalen Einführabschnitt mit einer Einführlänge L1, und einen proximalen Abschnitt, welche zusammen über eine Gesamtlänge L2 der Perfusionskanüle 10 vorgesehen sind. Die Perfusionskanüle 10 weist eine Kanülenwand 12 auf, welche im Bereich des proximalen Endes 10b einen Durchmesser D2 aufweist und im Bereich des distalen Endes 10a einen Durchmesser D1 aufweist. Der Durchmesser D1 ist kleiner als der Durchmesser D2. Die Perfusionskanüle 10 weist ein gestrichelt angedeutetes Sensorlumen 16 auf, in welchem eine elektrische Leitung geführt ist, über welche Ringelektroden 17.1, 17.2 elektrisch kontaktiert sind. Die Ringelektroden 17.1, 17.2 sind außen an der Kanülenwand 12 angeordnet. Die distale Elektrode 17.1 ist in einem Abstand x1 vom distalen Ende 10a angeordnet, wobei der Abstand x1 z.B. im Bereich von 4 cm liegt, und die proximale Elektrode 17.2 ist in einem Abstand x2 vom distalen Ende 10a angeordnet, so dass die Elektroden in einem Abstand x3 von etwa von 40 bis 60 mm zueinander angeordnet sind.

In der Fig. 2 ist eine Perfusionskanüle 10 mit einer Kanülenwand 12 gezeigt, wobei die Kanülenwand 12 eine zylindrische Innenmantelfläche 12a sowie eine zylindrische Außenmantelfläche 12b aufweist, und die zylindrische Innenmantelfläche 12a umgrenzt ein Perfusionslumen 13. Die Mantelflächen 12a, 12b sind konzentrisch um eine Mittenlängsachse M angeordnet. In die Kanülenwand 12 sind fünf Sensorlumina 16.1, 16.2, 16.3, 16.4, 16.5 integriert, die konzentrisch um die Mittenlängsachse M angeordnet sind. In einem ersten der Sensorlumina 16.1 ist ein EKG-Sensor 17 eingebettet, und in einem fünften der Sensorlumina 16.5 ist ein Drucksensor 19 eingebettet. Die Kanülenwand 12 weist eine Wandstärke d auf, die im Bereich von 0,75 mm liegt, und das erste Sensorlumen 16.1 weist einen Durchmesser d1 auf, der im Bereich von 0,25 mm liegt, und das fünfte Sensorlumen 16.5 weist einen Durchmesser d5 auf, der im Bereich von 0,55 mm liegt.

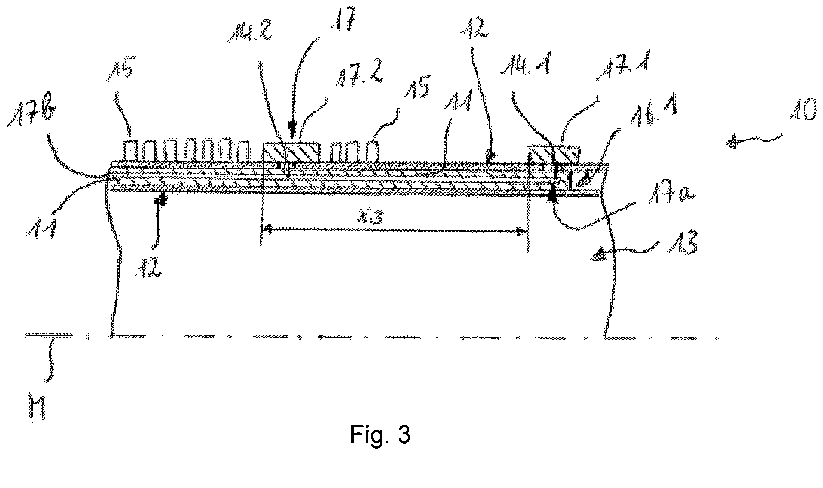

In der Fig. 3 ist eine Perfusionskanüle 10 mit einer Kanülenwand 12 gezeigt, die ein Perfusionslumen 13 umgrenzt. Die Perfusionskanüle 10 erstreckt sich entlang einer Mittenlängsachse M. In die Kanülenwand 12 ist ein Sensorlumen 16.1 für einen EKG-Sensor 17 integriert, wobei der EKG-Sensor 17 in dem Sensorlumen 16.1 in einer Einbettung 11 eingebettet ist, so dass ein distales Ende 17a des EKG-Sensors 17 von der Einbettung 11 umschlossen ist. Der EKG-Sensor 17 weist zwei Ringelektroden 17.1, 17.2 auf, welche in einem Abstand x3 voneinander um die Kanülenwand 12 angeordnet sind. Die Ringelektroden 17.1, 17.2 sind jeweils über Durchführungen 14.1, 14.2 mit einem elektrischen Leiter 17b des EKG-Sensors 17 verbunden. Die Ringelektroden 17.1, 17.2 bedecken die Durchführungen 14.1, 14.2. Ferner ist außen um die Kanülenwand 12 eine Drahtverstärkung 15 vorgesehen, welche ausgehend von einem proximalen Ende der Perfusionskanüle 10 bis zu der proximal angeordneten (zweiten) Ringelektrode 17.2 vorgesehen ist und wahlweise auch distal von der zweiten Ringelektrode 17.2 vorgesehen sein kann, wie dargestellt. Hierzu ist die Drahtverstärkung 15 bevorzugt aus zwei einzelnen Abschnitten gebildet.

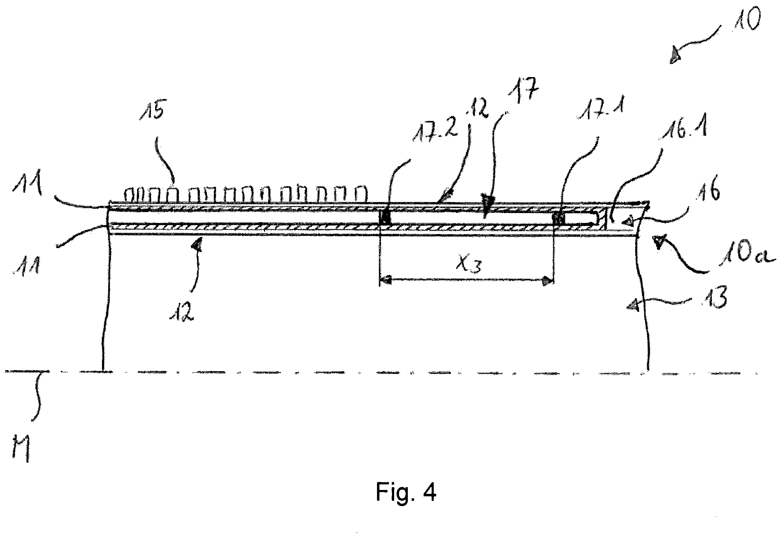

In der Fig. 4 ist eine Perfusionskanüle 10 gezeigt, welche im Unterschied zu der in Fig. 3 gezeigten Perfusionskanüle 10 keine Ringelektroden, sondern innerhalb eines Sensorlumens 16.1 angeordnete Elektroden 17.1, 17.2 eines EKG-Sensors 17 aufweist. Der EKG-Sensor 17 ist vollständig in einer Einbettung 11 angeordnet und (nur) gegenüber einem distalen Ende 10a der Perfusionskanüle 10 abgeschottet. Die Perfusionskanüle 10 weist daher keine Durchführungen auf, und eine Drahverstärkung 15 ist einstückig ausgeführt und durchgehend um eine Kanülenwand 12 gewickelt. Für eine Erläuterung der weiteren Bezugszeichen wird auf Fig. 3 Bezug genommen.

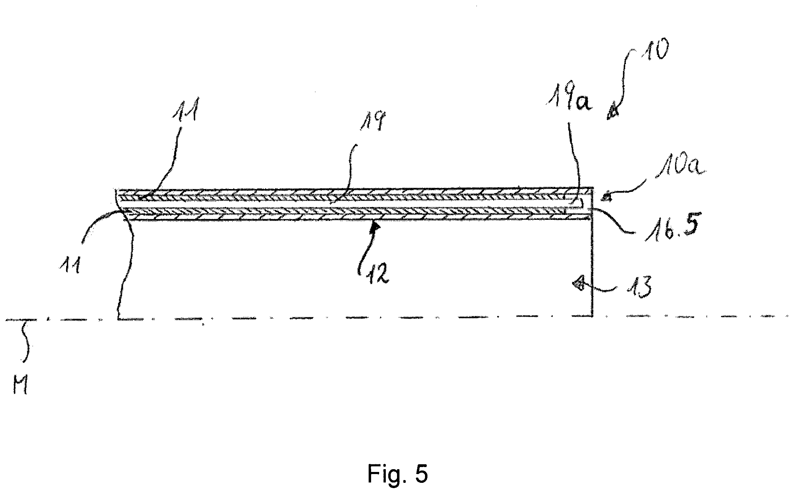

In der Fig. 5 ist eine Perfusionskanüle 10 mit einem Perfusionslumen 13 und mit einem Sensorlumen 16.5 gezeigt, in welchem ein Drucksensor 19 in einer Einbettung 11 innerhalb einer Kanülenwand 12 eingebettet ist, wobei ein distales Ende 19a des Drucksensors 19 in distaler Richtung aus der Einbettung 11 im Bereich eines distalen Endes 10a der Perfusionskanüle 10 hervorsteht. Hierdurch kann eine Druckmessung auf faseroptische Weise erfolgen. Die Perfusionskanüle 10 kann zusätzlich zu dem Sensorlumen 16.5 bzw. dem Drucksensor 19 wie in den Fig. 3 und 4 gezeigt ein Sensorlumen bzw. einen EKG-Sensor aufweisen.

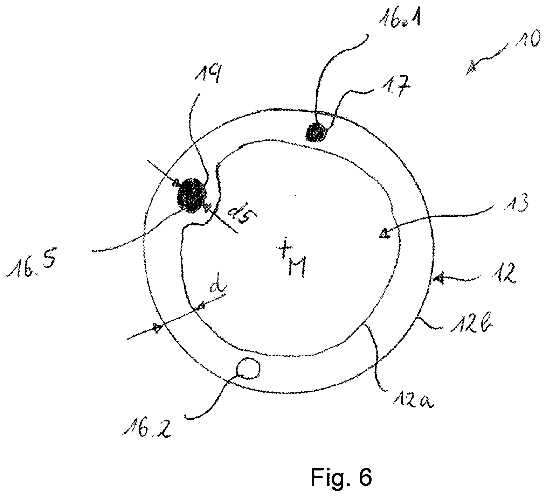

In der Fig. 6 ist eine Perfusionskanüle 10 mit einer Kanülenwand 12 gezeigt, wobei die Kanülenwand 12 eine Innenmantelfläche 12a sowie eine zylindrische Außenmantelfläche 12b aufweist, und die Innenmantelfläche 12a umgrenzt ein Perfusionslumen 13. Die Mantelflächen 12a, 12b sind konzentrisch um eine Mittenlängsachse M angeordnet. In die Kanülenwand 12 sind drei Lumina 16.1, 16.2, 16.5 integriert, ein erstes Sensorlumen 16.1 mit einem darin angeordneten EKG-Sensor 17 und ein fünftes Sensorlumen 16.5 mit einem darin angeordneten Drucksensor 19 sowie mit einem freien Arbeitslumen 16.2, in welchem bei Bedarf wahlweise eine Arbeitssonde (nicht dargestellt) anordenbar ist. Durch die Anordnung des fünften Sensorlumens 16.5 in einem nach innen hervorstehenden Teil der Kanülenwand 12 ist eine Wandstärke d der Kanülenwand 12 in Bezug auf einen Durchmesser d5 des fünften Sensorlumens 16.5 vergleichsweise dünn ausgeführt, insbesondere im Hinblick auf eine gute Flexibilität der Perfusionskanüle 10, selbst wenn ein größeres Sensorlumen oder Arbeitslumen erforderlich sein sollte.

### Bezugszeichenliste

- 1: System, insbesondere Lungen-Unterstützungssystem
- 10: Perfusionskanüle
- 10a: distales Ende der Perfusionskanüle
- 10b: proximales Ende der Perfusionskanüle
- 11: Einbettung
- 12: Kanülenwand
- 12a: innere Seite der Kanülenwand, insbesondere Innenmantelfläche
- 12b: äußere Seite der Kanülenwand, insbesondere Innenmantelfläche
- 13: Perfusionslumen
- 14.1: (erste) Durchführung durch Kanülenwand
- 14.2: (zweite) Durchführung durch Kanülenwand
- 15: Drahtverstärkung
- 16: Lumen
- 16.1: (erstes) Lumen
- 16.2: (zweites) Lumen
- 16.3: (drittes) Lumen
- 16.4: (viertes) Lumen
- 16.5: (fünftes) Lumen
- 17: Sensoreinrichtung, insbesondere EKG-Sensor
- 17a: distales Ende der Sensoreinrichtung
- 17b: elektrischer Leiter
- 17.1: (erste) Elektrode, z.B. Ringelektrode
- 17.2: (zweite) Elektrode, z.B. Ringelektrode
- 18: elektrische Verbindung zu einer Auswerteeinheit
- 19: weitere Sensoreinrichtung, insbesondere Drucksensor
- 19a: distales Ende der weiteren Sensoreinrichtung

- 20: Auswerteeinheit

- 30: Herz eines Patienten
- 31: Hauptschlagader (Aorta)
- 31a: Aortenbogen

- d: Breite der Kanülenwand in radialer Richtung (Wanddicke)
- d1: Durchmesser des ersten Lumens
- d5: Durchmesser des fünften Lumens

- D1: Außendurchmesser der Perfusionskanüle am distalen Ende
- D2: Außendurchmesser der Perfusionskanüle am proximalen Ende

- M: Mittenlängsachse der Perfusionskanüle

- L1: Einführlänge der Perfusionskanüle
- L2: Gesamtlänge der Perfusionskanüle

- x1: Abstand einer ersten Elektrode vom distalen Ende
- x2: Abstand einer zweiten Elektrode vom distalen Ende
- x3: Abstand der Elektroden zueinander

## Patentansprüche

1. Perfusionskanüle (10) zum Legen eines Zugangs, mit einer Kanülenwand (12) und einem von der Kanülenwand (12) umgrenzten Perfusionslumen (13), in welchem ein Fluidstrom die Perfusionskanüle (10) durchströmen kann; wobei die Kanülenwand (12) mit einer zumindest annähernd zylindrischen inneren Seite einer umlaufenden Innenmantelfläche (12a) und einer zumindest annähernd zylindrischen äußeren Seite einer umlaufenden Außenmantelfläche (12b) ausgebildet ist, wobei beide Mantelflächen (12a, 12b) konzentrisch angeordnet sind und
wobei die Perfusionskanüle (10) ferner wenigstens ein Sensorlumen (16.1, 16.2, 16.3, 16.4, 16.5) und wenigstens eine Sensoreinrichtung (17, 19) umfasst, welche zumindest teilweise in dem Sensorlumen (16.1, 16.2, 16.3, 16.4, 16.5) angeordnet ist,
**dadurch gekennzeichnet, dass**
das wenigstens eine Sensorlumen (16.5) in einem nach innen hervorstehenden Teil der Kanülenwand (12) angeordnet ist, deren Wandstärke (d), abgesehen von dem nach innen hervorstehenden Teil der Kanülenwand (12), in Bezug auf einen Durchmesser (d5) des Sensorlumens vergleichsweise dünn ausgebildet ist, und dass das wenigstens eine Sensorlumen (16.5) der Sensorlumina (16.1, 16.2, 16.3, 16.4, 16.5) als Sensoreinrichtung einen Drucksensor (19) umfasst.

2. Perfusionskanüle (10) nach Anspruch 1, **dadurch gekennzeichnet, dass** das Sensorlumen (16.1, 16.2, 16.3, 16.4, 16.5) in radialer Richtung seitlich, nach innen in das Perfusionslumen (13) hervorstehend an der Kanülenwand (12) angeordnet ist.

3. Perfusionskanüle (10) nach Anspruch 1 oder 2, **dadurch gekennzeichnet, dass** die Perfusionskanüle (10) eine Einführlänge (L1) von mehr als 15 cm, bevorzugt mehr als 20 cm, weiter bevorzugt mehr als 25 cm, insbesondere im Bereich von 25 bis 50 cm aufweist.

4. Perfusionskanüle (10) nach einem der vorhergehenden Ansprüche, **dadurch gekennzeichnet, dass** die Perfusionskanüle (10) im Bereich eines distalen Endes (10a) der Perfusionskanüle (10) einen Außendurchmesser zwischen 5/3 mm (5 French) und 35/3 mm (35 French), bevorzugt zwischen 4mm (12 French) und 25/3 mm (25 French) aufweist.

5. Perfusionskanüle (10) nach einem der vorhergehenden Ansprüche, **dadurch gekennzeichnet, dass** wenigstens eine zumindest teilweise in einem Sensorlumen (16.1) angeordnete Sensoreinrichtung (17) als EKG-Sensor ausgebildet ist und vorzugsweise das (EKG-)Sensorlumen (16.1) einen Durchmesser (d5) zwischen 0,05 und 2 mm, bevorzugt 0,2 und 0,5 mm aufweist.

6. Perfusionskanüle (10) nach einem der vorhergehenden Ansprüche, **dadurch gekennzeichnet, dass** wenigstens eine der wenigstens einen Sensoreinrichtung (17) als EKG-Sensor ausgebildet ist und eine Ringelektrode (17.1, 17.2) aufweist, die außen um die Kanülenwand (12) angeordnet ist und über eine Durchführung (14.1, 14.2) in der Kanülenwand (12) mittels eines in dem entsprechenden Sensorlumen (16.1) geführten elektrischen Leiters (17b) elektrisch kontaktiert ist.

7. Perfusionskanüle (10) nach einem der vorhergehenden Ansprüche, **dadurch gekennzeichnet, dass** wenigstens eine der wenigstens einen Sensoreinrichtung (17) als EKG-Sensor mit zwei innerhalb des Sensorlumens (16.1) angeordneten Elektroden (17.1, 17.2) ausgebildet ist.

8. Perfusionskanüle (10) nach Anspruch 1, **dadurch gekennzeichnet, dass** der Drucksensor (19) als faseroptischer Drucksensor ausgebildet ist.

9. Perfusionskanüle (10) nach Anspruch 8, **dadurch gekennzeichnet, dass** der faseroptische Drucksensor ein distales Ende (19a) aufweist, welches in einem Abstand von maximal 5 cm, bevorzugt maximal 3 cm, weiter bevorzugt maximal 1 cm von einem distalen Ende (10a) der Perfusionskanüle (10) angeordnet ist.

10. Perfusionskanüle (10) nach einem der vorhergehenden Ansprüche, **dadurch gekennzeichnet, dass** wenigstens eine der wenigstens einen Sensoreinrichtung (17, 19) fluiddicht im entsprechenden Sensorlumen (16.1, 16.2, 16.3, 16.4, 16.5) eingebettet ist, insbesondere im Bereich eines distalen Endes (17a, 19a) der Sensoreinrichtung (17, 19).

11. Perfusionskanüle (10) nach einem der vorhergehenden Ansprüche, **dadurch gekennzeichnet, dass** die Perfusionskanüle (10) eine Drahtverstärkung (15) aufweist, die vorzugsweise radial außen von dem wenigstens einen Sensorlumen (16.1, 16.2, 16.3, 16.4, 16.5) angeordnet ist.

12. Perfusionskanüle (10) nach einem der vorhergehenden Ansprüche, **dadurch gekennzeichnet, dass** die Perfusionskanüle (10) mit einer Schicht Polyurethan überzogen ist.

13. Perfusionskanüle (10) nach einem der vorhergehenden Ansprüche, **dadurch gekennzeichnet, dass** die Perfusionskanüle (10) aus Silikon, Silikon-Polyurethan Copolymer, PVC, Polyurethan und/oder nichtrostendem VA-Stahl besteht.

14. Perfusionskanüle (10) nach einem der vorhergehenden Ansprüche, **dadurch gekennzeichnet, dass** die Perfusionskanüle (10) ein freies Arbeitslumen (16.2, 16.3, 16.4) aufweist, in welchem eine Arbeitssonde anordenbar ist.

15. System (1), insbesondere Kreislauf-Unterstützungssystem, mit einer Perfusionskanüle (10) nach einem der vorhergehenden Ansprüche für einen arteriellen Zugang und mit einer Drainagekanüle sowie mit einer Auswerteeinheit (20) zum Auswerten von Messwerten zumindest einer der jeweils wenigstens einen Sensoreinrichtung (17, 19) der Perfusionskanüle (10).

## Claims

1. A perfusion cannula (10) for placement of an access, having a cannula wall (12) and a perfusion lumen (13) which is surrounded by the cannula wall (12) and in which a fluid flow can flow through the perfusion cannula (10);
wherein the cannula wall (12) is formed by an at least approximately cylindrical inner surface of a circumferential inner sleeve surface (12a) and by an at least approximately cylindrical outer surface of a circumferential outer sleeve surface (12b), wherein the sleeve surfaces (12a, 12b) are both arranged concentrically, and
wherein the perfusion cannula (10) furthermore comprises at least one sensor lumen (16.1, 16.2, 16.3, 16.4, 16.5) and at least one sensor device (17, 19), which is arranged at least partially in the sensor lumen (16.1, 16.2, 16.3, 16.4, 16.5), **characterized in that**
at least one sensor lumen (16.5) is arranged in an inwardly protruding part of the cannula wall (12), the wall thickness (d) of which is of a comparatively thin construction in relation to a diameter (d5) of the sensor lumen, expect for the inwardly protruding part of the cannula wall(12),
and that at least one sensor lumen (16.5) of the sensor lumina (16.1, 16.2, 16.3, 16.4 and 16.5)comprises as a sensor device (17) a pressure sensor (19).

2. Perfusion cannula (10) according to Claim 1, **characterised in that** the sensor lumen (16.1, 16.2, 16.3, 16.4, 16.5) is arranged protruding inwardly into the perfusion lumen (13)laterally in the radial direction.

3. Perfusion cannula (10) according to Claim 1 or 2, **characterised in that** the perfusion cannula (10) has an insertion length (L1) of greater than 15 cm, preferably greater than 20 cm, more preferably greater than 25 cm, in particular in the range of 25 to 50 cm.

4. Perfusion cannula (10) according to one of the preceding claims, **characterised in that** the perfusion cannula (10) has an external diameter of between 5/3 mm (5 French) and 35/3 mm (35 French), preferably between 4 mm (12 French) and 25/3 mm (25 French), in the region of a distal end (10a) of the perfusion cannula (10).

5. Perfusion cannula (10) according to one of the preceding claims, **characterised in that** at least one sensor device (17), which is at least partially arranged in a sensor lumen (16.1), is constructed as an ECG sensor and the (ECG) sensor lumen (16.1) has preferably a diameter (d5) of between 0.05 and 2 mm, preferably 0.2 and 0.5 mm.

6. Perfusion cannula (10) according to one of the preceding claims, **characterised in that** at least one of the at least one sensor device (17) is constructed as an ECG sensor and has an annular electrode (17.1, 17.2) which is arranged externally around the cannula wall (12) and, by way of a feed through (14.1, 14.2) in the cannula wall (12), is electrically contacted by means of an electrical conductor (17b) guided in the corresponding sensor lumen (16.1).

7. Perfusion cannula (10) according to one of the preceding claims, **characterised in that** at least one of the at least one sensor device (17) is constructed as an ECG sensor with two electrodes (17.1, 17.2) arranged within the sensor lumen (16.1) .

8. Perfusion cannula (10) according to claim 1, **characterized in that** the pressure sensor (19) is constructed as a fibre optic pressure sensor.

9. Perfusion cannula (10) according to claim 8, **characterized in that** the fibre optic pressure sensor has a distal end (19a) which is arranged at a spacing of a maximum of 5 cm, preferably a maximum of 3 cm, more preferably a maximum of 1 cm from a distal end (10a) of the perfusion cannula (10).

10. Perfusion cannula (10) according to one of the preceding claims, **characterised in that** at least one of the at least one sensor device (17, 19) is embedded in fluid-tight manner in the corresponding sensor lumen (16.1, 16.2, 16.3, 16.4, 16.5), in particular in the region of a distal end (17a, 19a) of the sensor device (17, 19).

11. Perfusion cannula (10) according to one of the preceding claims, **characterised in that** the perfusion cannula (10) has a wire reinforcement (15) which is preferably arranged radially outside the at least one sensor lumen (16.1, 16.2, 16.3, 16.4, 16.5) .

12. Perfusion cannula (10) according to one of the preceding claims, **characterised in that** the perfusion cannula (10) is covered with a polyurethane coating.

13. Perfusion cannula (10) according to one of the preceding claims, **characterised in that** the perfusion cannula (10) is made of silicone, silicone-polyurethane copolymer, PVC, polyurethane and/or stainless VA steel.

14. Perfusion cannula (10) according to one of the preceding claims, **characterised in that** the perfusion cannula (10) has a free operating lumen (16.2, 16.3, 16.4) in which an operating probe can be arranged.

15. A system (1), in particular a circulation support system, having a perfusion cannula (10) according to one of the preceding claims for an arterial access and having a drainage cannula and having an evaluating unit (20) for evaluating measured values of at least one of the in each case at least one sensor device (17, 19) of the perfusion cannula (10).

## Revendications

1. Canule de perfusion (10) destinée à poser un accès et comportant une paroi de canule (12) et une lumière de perfusion (13) délimitée par la paroi de canule (12) et dans laquelle un flux de fluide peut s'écouler à travers la canule de perfusion (10) ; la paroi de canule (12) étant formée avec un côté intérieur au moins approximativement cylindrique d'une surface d'enveloppe intérieure circonférentielle (12a) et un côté extérieur au moins approximativement cylindrique d'une surface d'enveloppe extérieure circonférentielle (12b), les deux surfaces d'enveloppe (12a, 12b) étant disposées concentriquement et
la canule de perfusion (10) comprenant en outre au moins une lumière de capteur (16.1, 16.2, 16.3, 16.4, 16.5) et au moins un dispositif de détection (17, 19) qui est disposé au moins partiellement dans la lumière de capteur (16.1, 16.2, 16.3, 16.4, 16.5),
**caractérisée en ce que**
l'au moins une lumière de capteur (16.5) est disposée dans une partie faisant saillie vers l'intérieur de la paroi de canule (12), dont l'épaisseur de paroi (d), à part la partie faisant saillie vers l'intérieur de la paroi de canule (12), est comparativement mince par rapport à un diamètre (d5) de la lumière de capteur, et **en ce que** l'au moins une lumière de capteur (16.5) des lumières de capteur (16.1, 16.2, 16.3, 16.4, 16.5) comprend un capteur de pression (19) en tant que dispositif de détection.

2. Canule de perfusion (10) selon la revendication 1, **caractérisée en ce que** la lumière de capteur (16.1, 16.2, 16.3, 16.4, 16.5) est disposée latéralement dans la direction radiale, en faisant saillie vers l'intérieur dans la lumière de perfusion (13) sur la paroi de canule (12).

3. Canule de perfusion (10) selon la revendication 1 ou 2, **caractérisée en ce que** la canule de perfusion (10) a une longueur d'insertion (L1) supérieure à 15 cm, de préférence supérieure à 20 cm, de manière davantage préférée supérieure à 25 cm, en particulier comprise dans une plage allant de 25 à 50 cm.

4. Canule de perfusion (10) selon l'une des revendications précédentes, **caractérisée en ce que** la canule de perfusion (10) présente un diamètre extérieur compris entre 5/3 mm (5 charrière) et 35/3 mm (35 charrière), de préférence entre 4 mm (12 charrière) et 25/3 mm (25 charrière) dans la zone d'une extrémité distale (10a) de la canule de perfusion (10).

5. Canule de perfusion (10) selon l'une des revendications précédentes, **caractérisée en ce qu'**au moins un dispositif de détection (17) disposé au moins partiellement dans une lumière de capteur (16.1) est conçu comme un capteur d'électrocardiogramme et de préférence la lumière de capteur (d'ECG) (16.1) présente un diamètre (d5) compris entre 0,05 et 2 mm, de préférence entre 0,2 et 0,5 mm.

6. Canule de perfusion (10) selon l'une des revendications précédentes, **caractérisée en ce qu'**au moins un de l'au moins un dispositif de détection (17) est conçu comme un capteur d'électrocardiogramme et comporte une électrode annulaire (17.1, 17.2) qui est disposée à l'extérieur autour de la paroi de canule (12) et est mise en contact électrique par un passage (14.1, 14.2) dans la paroi de canule (12) au moyen d'un conducteur électrique (17b) passant dans la lumière de capteur correspondante (16.1).

7. Canule de perfusion (10) selon l'une des revendications précédentes, **caractérisée en ce qu'**au moins un de l'au moins un dispositif de détection (17) est conçu comme un capteur d'électrocardiogramme avec deux électrodes (17.1, 17.2) disposées dans la lumière de capteur (16.1).

8. Canule de perfusion (10) selon la revendication 1, **caractérisée en ce que** le capteur de pression (19) est conçu comme un capteur de pression à fibre optique.

9. Canule de perfusion (10) selon la revendication 8, **caractérisée en ce que** le capteur de pression à fibre optique présente une extrémité distale (19a) qui est disposée à une distance de maximum 5 cm, de préférence de maximum 3 cm, de manière davantage préférée de maximum 1 cm d'une extrémité distale (10a) de la canule de perfusion (10).

10. Canule de perfusion (10) selon l'une des revendications précédentes, **caractérisée en ce qu'**au moins un de l'au moins un dispositif de détection (17, 19) est incorporé de manière étanche dans la lumière de capteur correspondante (16.1, 16.2, 16.3, 16.4, 16.5), en particulier dans la zone d'une extrémité distale (17a, 19a) du dispositif de détection (17, 19).

11. Canule de perfusion (10) selon l'une des revendications précédentes, **caractérisée en ce que** la canule de perfusion (10) présente une armature en fil métallique (15) qui est de préférence disposée radialement à l'extérieur de l'au moins une lumière de capteur (16.1, 16.2, 16.3, 16.4, 16.5).

12. Canule de perfusion (10) selon l'une des revendications précédentes, **caractérisée en ce que** la canule de perfusion (10) est revêtue d'une couche de polyuréthane.

13. Canule de perfusion (10) selon l'une des revendications précédentes, **caractérisée en ce que** la canule de perfusion (10) est constituée de silicone, d'un copolymère de silicone-polyuréthane, de PVC, de polyuréthane et/ou d'acier inoxydable V1.

14. Canule de perfusion (10) selon l'une des revendications précédentes, **caractérisée en ce que** la canule de perfusion (10) présente une lumière de travail libre (16.2, 16.3, 16.4) dans laquelle une sonde de travail peut être disposée.

15. Système (1), en particulier système d'assistance circulatoire, comportant une canule de perfusion (10) selon l'une des revendications précédentes pour un accès artériel et une canule de drainage ainsi qu'une unité d'évaluation (20) pour évaluer les valeurs mesurées d'au moins l'un de l'au moins un dispositif de détection (17, 19) de la canule de perfusion (10).
